# EUROPEAN PATENT APPLICATION

(11) **EP 1 378 225 A1**
(43) Date of publication of application: **07.01.2004**
(21) Application number: 03386015.6
(22) Date of filing: 05.06.2003
(51) Int. Cl.: A61K 7/00

(54) **Method for rejuvenation of body and/or face by selecting biomolecules**

(30) Priority: 26.06.2002 GR 2002100312
(71) Applicant: DR. Kleanthous - Kentro Avaneoseos Ke Omorfian A.E., 14563 Kifisia (GR)
(72) Inventor: Tsiounis, Basilios, 14564 Kifissia,Athens (GR)

(57) **Abstract**

The biomolecular therapies are used in many ways at the daily clinical practice. Depending on the cases, several standardized biomolecules or forms with biomolecular mixtures of various origins, which are accepted by the body with good results and without side effects are applied by injection.
The invention refers to a new method of individualizing qualitatively and quantitatively the administered biomolecular forms, so, according to its application, that the administration of the biomolecular forms for the rejuvenation, prevention or/and therapy is possible, according to the ascertainable needs of each person that is subjected to it.
The new method consists in the definition, individualized quantitatively and qualitatively, of biomolecules that are necessary for the general rejuvenation of the body or/and face, in combination with factors that are necessary for the proper assessment of the body's biological damage of the body or/and the face.
With the analysis of specific parameters, during the new method essential organs of the body or/and face, neck and décolleté are rejuvenated biologically.
This fact takes place with the realization of a synthesis that is based on specific parameters, of a special individualized biomolecular mixture, which rejuvenates biologically the body or the face, the neck and décolleté as a whole.

The results of such a biomolecular rejuvenation according to the new method, regardless of the fact if it is applied to a man or a woman, are good health, well-being, and youthfulness.
According to the new method each person takes what exactly, from a therapeutic point of view, he/she needs, in order to have again his/her lost vitality and additionally to restore, as much as it is possible, the proper function of the organs that are defective. The body or/and the face is becoming, depending on each case, about 5-10 years biologically younger, with all the physical and mental consequences that this fact involves.
For example the body pressures from the everyday stress diminish, the fatigue is reduced, the mood is getting better, the resistance to the oppressive cases is increased and the mobility is improved. The results last so many years as the biological clock is getting back, but even though these years are passed, the biological precedence remains as the body is getting older naturally from the point that is reached after the therapy.

Taking into account that the specific biomolecules do not act toxically, are considered from the body as that they belong to the body and, according to the nobelist Linus Pauling, they act in an absolutely positive way for the specific invigoration of the same therapeutic powers of the body.
The therapies of the general rejuvenation are made independently, preventively or suppressively or for the maintenance, as well as on the side because they are not coming in contrast to any other treatment that is already applied or it is going to be applied.

## Description

The modern biological research has been shifted on the field of macromolecules since the various clinical researches come to the conclusion, that the process of aging and the most degenerative diseases and many other illnesses are directly connected with molecular - macromolecular disorders.
The eukaryotic cell is consists of the nucleus with the karyotheca, the cytosome with the organelles and the cell membrane.

The nucleus contains the nucleic acids, the cytosome and the organelles but also provides the suitable conditions for the cell activities while the cell membrane ensures, through specific mechanisms, according to the case, the cell communication with the environment.

The cell function is achieved by the entire combination and action control of the cytosome's and cell membrane's functions having as a result the continuous flowand the exchange of information with the intracellular and extracellular environment.

Important factor to the exchange and the modulation of the cell information and signals constitute the low molecular compounds with low molecular weight (almost 30kd) that are called biomolecules.
The biomolecules are natural buffer substances and metabolites.
The biological damage and the disorders of the specific action mechanism of the biomolecules reflect on disorders to the exchange of information and signals and contribute to the evolution and appearance of pathophysiological situations.
The result is the deficient cell function that is characterized by the deregulation of the cell cycle, the disorder of the cell metabolism and consequently the induced cell degeneration.
The associated aggravating factors lead to the disorder of the homeostasis and the creation of pathophysiological situations such as the various diseases (degenerative or non degenerative ones, benign or malignant neoplasms, the aging etc.

The impacts due to the time, act cumulatively having as a result the disorganization of the function system of the basic cell, structural materials which are the biomolecules of the cell.

At this point, we should stress the fact that a cell, when it is divided, transfers to its descendant, all of its good and bad characteristics.
Only in special cases of damage of genetic material, it can interfere and make them up. As regards the characteristics of the function of the cytosome, it continues to function exactly as its ancestor with the same characteristics, but it never stops making worse more and more, up to the next division and the cycle is continued with unimpaired tension. So, we finally have the chronic cell degeneration, which has as a result the cell senescence. This process will end up to some long-term situations, which are called pathophysiological situations.

Meaning situations, which are considered pathological ones in general, but they go with the deterioration of the physiological phenotype with other aggravating factors, such as the age and so they are estimated as normal (physiological).

The application of biomolecules, decelerates or / and supports the cell in order to reduce the deceleration that it went through, since their action is located in the cytosome. So, it gives a new impulse to the aggravating mechanisms giving the chance to the cells, on which is applied to function better.
The division of these cells, gives cells with better and more renewal biological characteristics, having a reverse process from this that we previously described.
So, the cell strengthens itself with the same powers and can be ready in order to cope with the assaults or/and the surcharges, which it goes through at this moment without having to do something important, but declining.

The new method consists in the definition of, individualized quantitatively and qualitatively, biomolecules that are necessary for the general rejuvenation of the body or/and face of each specific person, combined with factors that are needed for the proper assessment of the body's biological damage of its body or / and the face.
With the analysis of the parameters, essential organs of the body or/ and face, neck and décolleté rejuvenate biologically during the new method.
This fact takes place with the synthesis of an especially individualized biomolecular mixture, which rejuvenates biologically the body or the face, the neck and the décolleté as a whole.
Or, being based on specific parameters, the difference of the new method, relatively the up today applied practices, which do not bring about general rejuvenation of the body but only an incidental and partial rejuvenation of several organs and which use for every person indepedently and regardless of age, sex and the other personal peculiarities, either at the same standardized composition of a biomolecular preparation or/and mixture, or the same standardized composition of mixtures that are administrated in some cases lies exactly to the individualized composition of a biomolecular mixture of a general body or/and face rejuvenation, that is each time absolutely adapted qualitatively and quantitatively to the needs and peculiarities of each person lies exactly.

### METHODOLOGY

The new method consists in the definition of, individualized quantitatively and qualitatively, biomolecules that are necessary for the general rejuvenation of the body and face, combined with factors that are needed for the proper assessment of the biological damage of each specific body or / and the face.
With the analysis of parameters, according to the new method, the essential organs of the body or / and face, neck and décolleté rejuvenate biologically.
This fact takes place with the synthesis of an especially individualized biomolecular mixture, which rejuvenates biologically the body or the face, the neck and the décolleté as a whole.

During the General Body Rejuvenation, some of the essential organs of the body such as the heart, the reins, the liver, the spleen, the pancreas and the brain are rejuvenated. Then, for the preparation, during the new method, of the appropriate and individualized qualitatively and quantitatively biomolecular mixture, the following factors are analyzed.
- Calendar age
- Biological age
- Sex
- Lifestyle and life habits
- Detailed medical history
- Physical examination (clinical and laboratory control according to the medical history.)
- Mental state
- Environmental conditions

Thus, according to the prevailing point of view, the rejuvenation of a body would have focused on its essential organs and its main systems.
So, in all the applications that we have up to now, standardized biomolecules or mixtures of biomolecules are administrated, aiming to approach, as much as it is possible, the general rejuvenation of the body, through the rejuvenation of the parts of the body.
That means, that in different people, of various age and sex almost the same composition of the biomolecular mixture is administrated, so, from the one side, for every person who is subjected to such a treatment, there are several positive results of partial well being to his/her body, but from the other way these results are far from the basic goal, that is the general rejuvenation of his/her body.

### A New method. Example 1, for a man 72 years old and a woman 57 years old.

According to the new method, at first, especially the data of his / her calendar age and his/her biological age that shows the state of health, in relation with the aggravations that it has suffered due to several structural and functional modifications of the cells as well as of the body is taken into account.
So, according to specific parameters, the first part of the qualitative and quantitative composition of the individualized biomolecular mixture, that mainly concerns the heart, the liver, the loins, the brain in general, the pancreas and the muscles, is determined.

Further to, based on the sex, the detailed medical history and the physical examination, according to specific parameters, the second part of the qualitative and quantitative composition of the individualized biomolecular mixture is determined.
As far as our example is concerned, to the one man's mixture will be added biomolecules, in a specific analogy, from a special part of the brain (the case of enhancement of memory) or/and prostate (the case of prostatauxe), as to the other one woman's mixture, biomolecules for the bones and the spinal cord (the case of osteoporosis) will be added.
Accordingly, if these persons have a high blood pressure or a low blood pressure, at every individual mixture of biomolecules, the biomolecules will be added in special analogy, from a specific area of the placenta.
If, for instance, a man needs a stimulation of the immune system, the biomolecules that will be added from the thymus (gland), whereas if a woman has an intense stress and phobias, then, she should be administrated by special molecules from the pineal gland and the brain.
If one of the two people faces intense mental situations or/and he/she suffers from intense charges and troubles from his/her environment, then the atomic biomolecular mixture should be enriched by biomolecules from the pineal gland, the puitary gland and from the interbrain, but according to a definite analogy and always combined with the rest of its composition.

From all that are mentioned above, there is the conclusion that the definition of the factors mentioned in page 3 of the present, lines 20-28 that helps to the proper assessment of the biological damage, allows, according to the parameters of the new method, the preparation of a biomolecular mixture which qualitatively and quantitatively is absolutely adjusted to the total of the special and general needs of every person, getting, thus, the essential general rejuvenation of its body.

Due to this individualization, lies the big difference of the new method compared with the other practices, but also the significance and the success of the new method as for the general rejuvenation of the body, a fact that can be confirmed in any clinical application.

So, conclusively, it is mentioned, that the analysis and the combination of these factors shows the total individualized biological aggravation of the body, so, in relation to the necessary initial but also secondary organs, in combination and according to the special parameters of the new method, the suitable, according to the situation, biomolecular mixture for the General Biomolecular Body Rejuvenation is prepared qualitatively and quantitatively.

For the General Biomolecular Facial Rejuvenation (neck and décolleté), according to the new method, some of the essential organs of the body, in a typical case, that are renewed are the muscles of the face, the neck muscles and the décolleté ones, the skin and the conjunctive tissue of the face.
Further to, for the preparation of the suitable and individualized qualitatively and quantitatively biomolecular mixture the following factors are analyzed:
- Calendar age
- Biological age
- Sex
- Lifestyle and life habits
- Detailed special medical history
- Physical examination (clinical and laboratory control according to the medical history).
- Mental state
- Environmental conditions

The analysis and the combination of these factors, according to the new method, shows the total individualized aggravation of the face, the neck and the décolleté and in combination with the necessary basic organs of the area but also the body in general, according to the new method, is prepared qualitatively and quantitatively the appropriate, according to the case, biomolecular mixture for the General Biomolecular Rejuvenation of the face, the neck and the décolleté.

The up to this day applications for the rejuvenation of the face look a little bit or not a bit like the new method. They usually have an operative form (scalpel, laser, addition of collagen or other similar materials) and a different theory and facing. Besides, there are some methods that use biomolecules, but in a complete different way and always as a standardized administration which, de facto, does not include a guarantee of the results for the specific person in whom it will be applied. That means, that the methods that are applied and their goals, are far from the basic goal of the new method, that is the general rejuvenation of the face, neck and décolleté.

### A New method. Example 2, for a woman 45 years old and a woman 56 years old.

The cells of the skin are subject to an aging process, a fact that is reflected to their weakness to produce, qualitatively and quantitatively, the biomolecules that are necessary for the maintenance of their functional balance, having as a result the reduction of the renewal ability of the skin.
The disorder of the harmonious function of the metabolism, the reduced elimination of its waste substances, the loss in humidity and the diminution of collagen and elastin's production have as a result the skin to become thinner, less elastic and pale. The slackening becomes visible and follows the creation of wrinkles that are increased more and more as the time goes by.

According to the new method, especially the data of his/her calendar age and the biological age that shows the state of health is taken, at first, into consideration and especially that of the skin, in relation with the aggravations that they have suffered due to several structural and functional modifications of the cells of the area as well as of the body.
So, according to specific parameters, the first part of the qualitative and quantitative composition of the individualized biomolecular mixture, 7that mainly concerns the muscles of the face, neck and décolleté and the conjuctive tissue of the face, is determined.
Further to, given the sex, the detailed medical history and physical examination, according to specific parameters, the second part of the qualitative and quantitative composition of the individualized biomolecular mixture is determined. To our example, for instance, to the woman's mixture, who is in a premenopausal phase, will be added biomolecules for the normalization of her cycle, to the other one woman's mixture, who is in postmenopausal phase, will be added biomolecules for the general enhancement of her bones.

Accordingly, whether persons have an allergic predisposition or not, biomolecules would be added in the mixture for the equalization of the immune system.
If one of the two persons faces problems and has a complaint to the mouth cavity and mainly in her gums, then the individual biomolecular mixture, according to a definite analogy, and always in combination with the rest of its composition, should be enriched with biomolecules for facing periodontitis.

From all that are mentioned above, it is concluded that the definition of the factors that are mentioned in page 5 of the present paper, lines 1-9 that helps to the proper assessment of the biological damage, allows, according to the parameters of the new method, the preparation of a biomolecular mixture which, qualitatively and quantitatively, has to be absolutely adjusted to the total of the special and general needs of every person, achieving, thus, the essential general rejuvenation of the face, neck and décolleté.

Due to this individualization, lies the big difference of the new method in comparison with the other practices, but also the significance and the success of the new method as for the general rejuvenation of the face, neck, and décolleté, a fact that can be confirmed in any clinical application.

So, conclusively, it is mentioned, that the analysis and the combination of these factors shows the total individualized biological aggravation of the body, but especially of the face, neck and décolleté as well, so in relation to the necessary essential but also secondary organs, in combination and according to the special parameters of the new method, the suitable, according to the situation, biomolecular mixture for the General Biomolecular Rejuvenation of the Face, neck and décolleté is prepared qualitatively and quantitatively.

The new method of the face, neck and décolleté rejuvenation, among other things, has a completely different way of application. At the first phase of application, the biomolecules that compose, according to the above, the formed qualitatively and quantitatively individualized mixture, are administered hypodermically with a predetermined series and to specific points according to the new method.
The second phase, through the help of ultrasounds and following a specific process, the qualitative and quantitative feeding of all the cell layers up to the connective tissue with the necessary biomolecules is achieved.

With the administration of the relevant biomolecules through the new method is achieved gradually, the normalization of the metabolism, the increase of the aqueous reserve and also the increase of the production of collagen and elastin, in order to have, finally, a general active and natural rejuvenation of the face, neck and décolleté skin from the same forces of the body.

The visible positive results and their prolonged maintenance exist in all people of any age, to an extent that depends on the state of the skin of each one.

According to the above and in combination with the rejuvenation of the necessary basic, but also secondary organs as for the body, the face, the neck and the décolleté, it is reasonable that, according to the new method, the suitable, as the case may be, the biomolecular mixtures can be prepared qualitatively and quantitatively for the simultaneous General Biomolecular Body Rejuvenation and the General Biomolecular Rejuvenation of the Face, Neck and Décolleté.
It is self-evident that a general rejuvenation of the face, neck, and décolleté is always better when it takes place at the same time with a general body rejuvenation.

Also, it is mentioned, that the new method can be applied for the management of several diseases, with special biomolecular therapies in combination with a general rejuvenation and regardless of any other relative treatment that is already applied.
All the reasoning and the methodology that is followed regarding the general body rejuvenation, it is in force and is followed in the case of a specific disease, too.

In such a case, according to the new method, not only the data of the calendar and biological age, the sex, the detailed medical history, the physical examination, the mental condition and the influences from the environment are taken into consderation, but the pathological and pathophysiological symptoms that show the state of health and of the specific problem, in combination with the aggravations that went through due to several structural and functional alternations of the cells at the affected area and by extension of all the body as well.
So, according to specific parameters, the qualitative and quantitative composition of the individualized biomolecular mixture concerns the affected organ along with all the other organs that collaborate directly with it and the total of the body.
In a person who has a heart disease, for e.g., his/her individualized biomolecular form will contain all the necessary biomolecules for the general rejuvenation of his/her body and at the same time in special analogy, according to the parameters of the new method, of special biomolecules for the heart, the arteries, the reins and the liver.

From all that are mentioned above, there is a conclusion that the definition of the factors that are mentioned in page 3 of the present, lines 20-28 that helps to the proper assessment of the biological damage, in a combination with the usual symptomatic and diagnostic factors that concern a specific health problem, allows, according to the parameters of the new method, the preparation of a biomolecular mixture which qualitatively and quantitatively has to be absolutely adjusted to the total of the special and the general needs, as well as to the illness of each person, achieving, thus, the essential general rejuvenation of his/her body and the cure or at least the improvement of the clinical status of his/her case.

In this individualization, lies the big difference of the new method in comparison with the other practices, but also the significance and the success of the new method as to the general rejuvenation of the body and the confrontation of several diseases, a fact that can be confirmed in various clinical applications.

## Claims

1. A new method that consists in the definition, individualized quantitatively and qualitatively, of biomolecules that are necessary for the general revitalization of each body or/and face, neck and décolleté with agents that are necessary for the proper assessment of the body's biological damage. With the analysis of the parameters, essential organs of the body or/ and face, neck and décolleté rejuvenate biologically during the new method.
This fact takes place with the synthesis of an especially individualized biomolecular mixture, which rejuvenates biologically the body or the face, the neck and the décolleté as a whole. During the General Body Rejuvenation, people over 30 years old are rejuvenated from the essential organs of the body such as the heart, the reins, the liver, the spleen, the pancreas and the brain. Then, for the preparation, during the new method, of the appropriate and individualized qualitatively and quantitatively biomolecular mixture, the following factors are analyzed.
- Calendar age
- Biological age
- Sex
- Lifestyle and life habits
- Detailed special medical history
- Physical examination (clinical and laboratory control according to the medical history).
- Mental state
- Environmental conditions

2. A new method that consists in the definition, individualized quantitatively and qualitatively, of biomolecules that are necessary for the general revitalization of each body or/and face, neck and décolleté with agents that are necessary for the proper assessment of the body's biological damage. With the analysis of the parameters, essential organs of the body or/ and face, neck and décolleté rejuvenate biologically during the new method. This fact takes place with the synthesis of an especially individualistic biomolecular mixture, which rejuvenates biologically the body or / and the face, the neck and the décolleté as a whole.
During the General Body Rejuvenation, of the face, neck and décolleté, according to the new method, some of the essential organs of the body, in a typical case, that rejuvenate are the muscles of the face, the neck and the décolleté, the skin and the conjunctive tissue of the face. Then, for the preparation, according to the new method, of the appropriate and individualized qualitatively and quantitatively biomolecular mixture, the following factors are analyzed.
- Calendar age
- Biological age
- Sex
- Lifestyle and life habits
- Detailed medical history
- Physical examination (clinical and laboratory control according to the medical history.)
- Mental state
- Environmental conditions

3. Application, exploitation and commercial development of the method that concerns the definition of the damage of the essential organs of the body, according to the claim 1.

4. Application, exploitation and commercial development of the method that concerns the definition of the general damage of the body, according to the claim 1.

5. Application of the method, exploitation and commercial development that concerns the definition of the damage of the essential organs of the face, neck and décolleté, according to the claim 2.

6. Application, exploitation and commercial development of the method that concerns the definition of the general damage of the face, neck and décolleté, according to the claim 2.

7. Application, exploitation and commercial development of the method that concerns the definition and composition of the necessary, for the administration, biomolecules for the damage of the essential organs of the body, according to the claim 1.

8. Application, exploitation and commercial development of the method that concerns the definition and composition of the mixture of the necessary, for the administration, biomolecules for the general damage of the body, according to the claim 1.

9. Application, exploitation and commercial development of the method that concerns the definition and composition of the necessary, for the administration, biomolecules for the damage of the essential organs of the face, neck and décolleté, according to the claim 2.

10. Application, exploitation and commercial development of the method that concerns the definition and composition of the necessary, for the administration, biomolecules for the general damage of the face, neck and décolleté, according to the claim 2.

11. Application, exploitation and commercial development of the method that concerns the definition and composition of the biomolecular forms' administration, mixtures, treatments and therapies for the damage of the body, face, neck and décolleté, according to the claims 1 and 2.

12. Application, exploitation and commercial development of the method that concerns the biomolecular rejuvenation, the biomolecular revitalization, the body care - body rejuvenation - the revitalization of the body, face, neck and décolleté, according to the claims 1 and 2.

13. Application, exploitation and commercial development of the method that concerns the effect of the applications and therapies - treatment, well being, beauty rejuvenation and care of the body, face, neck and décolleté for the better quality of life, according to the claims 1 and 2.

14. Application, exploitation and commercial development of the method that concerns the biomolecular general and special rejuvenation and therapy of the body, face, neck and décolleté for the better quality of life, according to the claims 1 and 2.
